# NEUE EUROPÄISCHE PATENTSCHRIFT

(11) **EP 1 523 299 B2**
(45) Veröffentlichungstag und Bekanntmachung des Hinweises auf die Entscheidung über den Einspruch: **13.06.2012**
(45) Hinweis auf die Patenterteilung: 24.05.2006
(21) Anmeldenummer: 03765010.8
(22) Anmeldetag: 16.07.2003
(51) Int. Cl.: A61K 8/36, A61K 8/81, A61K 8/92, A61Q 1/10

(54) **FEUCHTIGKEITSBESTÄNDIGE MASCARA-ZUSAMMENSETZUNG**
MOISTURE-RESISTANT MASCARA COMPOSITION
COMPOSITION DE MASCARA RESISTANTE A L'HUMIDITE

(30) Priorität: 18.07.2002 DE 10233288
(43) Veröffentlichungstag der Anmeldung: 20.04.2005
(73) Patentinhaber: Coty B.V., 2031 CC Haarlem (NL)
(72) Erfinder: LOGINOVA, Yelena, Bronx, NY 10467 (US); FARER, Alan, Kinnelon, NJ 07405 (US); MACCHIO, Ralph, Sparta, NJ 07971 (US)
(74) Vertreter: Walter, Wolf-Jürgen
(86) Internationale Anmeldenummer: PCT/EP2003/007751
(87) Internationale Veröffentlichungsnummer: WO 2004/009043

(56) Entgegenhaltungen:
- EP-A- 0 557 196
- WO-A-02/30368
- WO-A-99/20230
- DE-A1- 10 033 527
- FR-A- 2 659 011
- US-A- 5 874 072

## Beschreibung

Die Erfindung betrifft eine Mascara-Zusammensetzung mit erhöhter Feuchtigkeits- und Abriebbeständigkeit.

Es ist bekannt, Polyvinylpyrrolidon wegen seiner guten Haftungseigenschaften an keratinischen Substraten als Filmbildner in Haarsprays oder Mascaras einzubringen. Als teilweise öllösliches aber insbesondere wasserlösliches Material hat Polyvinylpyrrolidon hygroskopische Eigenschaften, die die Stabilität des Eridproduktes nach seinem Auftragen auf Haare oder Wimpern verringern und zum Verschmieren des Mascaras und damit zur Verkürzung der Tragezeit beiträgt.

Die US-A-6255421 beschreibt ein Polymerisationsverfahren zur Herstellung eines Vinylpolymeren aus Vinylmonomeren wie u.a. Vinylpyrrolidon oder Comonomere mit Vinylacatat, bei dem ein mit Öl gequollenes Polymerpulver hergestellt werden kann, das mit Wasser zur Bildung einer gleichmäßigen flüssigen Emulsion verarbeitet werden kann. Hierzu sind spezielle Verfahrensschritte notwendig.

Der Erfindung liegt die Aufgabe zugrunde, eine Mascara-Zusammensetzung mit verbesserter Feuchtigkeitsbeständigkeit und Abriebbeständigkeit sowie ausgezeichneter Haftung bereitzustellen.

Erfindungsgemäß ist die Mascara-Zusammensetzung gekennzeichnet durch einen Komplex, bestehend aus
0,1 bis 10 Gew-% eines wasserlöslichen Polymeren, ausgewählt unter Polyvinylpyrrolidon, Vinylacetat/Vinylpyrrolidon-Copolymeren und Gemischen davon,
0,5 bis 10 Gew-% Stearinsäure und
1 bis 40 Gew-% eines Wachses oder Wachsgemisches,
wobei der Komplex als Ölphase in einer wäßrigen Phase emulgiert vorliegt.

Bei dem erfindungsgemäßen Mascara liegt das wasserlösliche Polymere nicht wie bisher bei bekannten Präparaten in der Wasserphase gelöst vor, sondern es befindet sich in der Ölphase und bildet zusammen mit Wachsen und Stearinsäure einen Komplex, der unübliche physikalische Eigenschaften hat. Dieser Komplex hat eine sehr gute Plastizität im Vergleich mit dem Wachsgemisch oder Wachs, die üblicherweise eingesetzt werden. Im Vergleich mit dem einfachen Polymerfilm auf der Oberfläche der Haare/Wimpern hat der Komplex eine deutlich erhöhte Feuchtigkeitsbeständigkeit und haftet sehr gut auf den Wimpern.

Da Mascara-Zusammensetzungen eine halbflüssige (oder halbfeste) Substanz darstellen, sind bei jeder Mascara-Anwendung die Verteilungs- und das Verfestigungseigenschaften der Produktes Merkmale von wesentlicher Bedeutung. Mit der vorliegenden Erfindung wird ein Produkt bereitgestellt, bei dem eine ausreichende Zeit zur Verteilung des Mascara auf den Wimpern zur Verfügung steht und das Ziel einer Wimpernverdickung, Wimpernverlängerung und Wimperndefinierung ohne unerwünschte negative Erscheinungsformen gewährleistet ist. Ermöglicht wird dies dadurch, daß die Einbeziehung der wasserlöslichen Polymeren in die Ölphase die Möglichkeiten für die Formulierung dieses Produktes mit z.B. wimpernverdickenden Eigenschaften verbessern. Eine verbesserte Abriebbeständigkeit und die Minimierung der hygroskopischen Eigenschaften der wasserlöslichen Polymeren sind das wesentliche Merkmal des erfindungsgemäßen Produktes.

Es wurde weiterhin gefunden, daß durch die Zusammenführung von PVP oder dem VA/VP-Copolymeren (im folgenden: PVP) mit dem Wachs und Stearinsäure und dem Eintrag starker mechanischer Kräfte bei Temperaturen im Schmelzbereich der Wachse bzw. 70-75 °C ein sehr homogener und stabiler Komplex entsteht, der zwar kein Komplex auf Basis chemischer Bindungen ist, jedoch ein sehr stabiles kolloidales System darstellt, aus dem PVP auch bei späterem Kontakt mit der wässrigen Phase nicht oder nur in ganz geringem Maße austritt. Das bedeutet, die zwischenmolekularen Kräfte sind so stark, dass sie eine Abwanderung von PVP im wesentlichen verhindern.

Versuche mit 4 Gew-% PVP in Wachs bzw. in Wasser haben gezeigt, daß nach Abdampfen des gesamten Wassers bei PVP/Wachs 5 Gew-% Feststoff erhalten wurden, was durch die Erhöhung um 1 Gew-% bereits darauf hindeutet, daß eine strukturelle Veränderung stattgefunden hat, während man bei PVP/Wasser die eingesetzten 4 Gew-% Feststoffe erhielt. Weiterhin wurde gefunden, dass bei dem erfindungsgemäßen Komplex große glänzende, schwach gelbliche, härtere Kristalle als Rückstand mikroskopisch erkennbar waren, während im anderen Falle bei in Wasser solubilisiertem PVP nur sehr kleine Teilchen bildeten.

Der wäßrigen Phase können je nach Löslichkeit und Mischbarkeit weitere Hilfsstoffe, Trägerstoffe und Wirkstoffe oder Gemische davon zugesetzt werden.

Bevorzugte Gehalte an Stearinsäure liegen im Bereich von 2 bis 8 Gew-%. Bevorzugte Gehalte an Wachs, das vorzugsweise ein Gemisch mehrere Wachse ist, liegen im Bereich von 10 bis 28 Gew-%.

Das Verhältnis von wasserlöslichem Polymeren bzw. Copolymeren zu Stearinsäure kann für das Polymere 0,1 bis 50 Gew-% und für die Stearinsäure 50 bis 99,1 Gew-% betragen.

Pigmente, Pigmentgemische oder Pulver mit pigmentartiger Wirkung, worunter auch solche mit Perlglanz-Effekt zu verstehen sind, können zum Beispiel umfassen Eisenoxide, natürliche Aluminiumsilicate wie Ocker, Titan(di)oxid, Glimmer, Kaolin, manganhaltige Tone wie Umbra und roter Bolus, Calciumcarbonat, Talkum, Glimmer-Titanoxid, Glimmer-Titanoxid-Eisenoxid, Wis-mutoxychlorid, Nylonkügelchen, Keramikkügelchen, expandierte und nichtexpandierte synthetische Polymerpulver, Cellulose/Rayon, Teflon, Baumwollfasern, pulverförmige natürliche organische Verbindungen wie gemahlene Festalgen, gemahlene Pflanzenteile, verkapselte und unverkapselte Getreidestärken sowie Glimmer-Titanoxid-organischer Farbstoff.

Zu Antioxidationsmitteln gehören Vitamine wie Vitamin C; Folsäure und deren Derivate; Aminosäuren, wie Histidin, Glycin, Tyrosin, Tryptophan und Derivate davon; Harnsäure und Derivate davon; α-Hydroxysäuren wie Citronensäure, Milchsäure, Apfelsäure; sowie Granatapfelextrakte.

Mascaras mit der erfindungsgemäßen Zusammensetzung können als O/W- oder W/O-Emulsionen vorliegen. Geeignete Emulgatoren für O/W-Emulsionen sind beispielsweise Anlagerungsprodukte von 2-30 Mol Ethylenoxid an lineare C₈-C₂₂-Fettalkohole, an C₁₂-C₂₂₋Fettsäuren und an C₈-C₁₅-Alkylphenole; C₁₂-C₂₂-Fettsäuremono- und - diester von Anlagerungsprodukten von 1-30 Mol Ethylenoxid an Glycerin. Bevorzugte Emulgatoren sind z.B. solche wie Polysorbate 20.

Copolymere von Polysiloxan-Polyalkylpolyethern sind mögliche Emulgatoren. Ein bevorzugter Emulgator ist Cetyl Dimethicone Copolyol.

Es ist weiterhin vorteilhaft, den erfindungsgemäßen Zusammensetzungen entsprechende wasser- lösliche UVA- oder UVB-Filter zuzusetzen.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Die Wachse können ausgewählt werden unter natürlichen pflanzlichen Wachsen, tierischen Wachsen, natürlichen und synthetische Mineralwachsen und synthetischen Wachsen. Dazu gehören beispielsweise Carnaubawachs, Candellilawachs, Ozokerit, Bienenwachs, Montanwachs, Wollwachs, Ceresin, Mikrowachse, Hartparaffin, Petrolatum, Silicone, Polyethylenglycol- oder -glycolesterwachse. mikrokristalline Wachse. Gemische mehrerer Wachse sind bevorzugt, wie z.B. Paraffinwachs, Carnaubawachs und Bienenwachs.

Es wurden Vergleichsversuche durchgeführt, z.B ein Feuchtigkeitstest gegenüber handelsüblichem PVP/VA Copolymer (Luviskol® VA64W) unter Verwendung einer Feuchtigkeitskammer bei 32 °C, 80% relativer Luftfeuchtigkeit für 24 Std.

Der Wachskomplex aus: Bienenwachs/Stearinsäure/Carnaubawachs/-Candelillawachs und PVP im Verhältnis 6:5:2:1:4 und handelsübliches PVP/VA Copolymer wurden auf ein inertes Substrat als 200 µm Film bis zur vollständigen Trocknung aufgetragen. Dann wurden die Probekörper für 24 Stunden in die Feuchtigkeitskammer gebracht.

Das Gewicht der Probekörper wurde in der folgenden Reihenfolge ermittelt:
- Wiegen des Substrates
- Wiegen des feuchten Films
- Wiegen des trockenen Films vor der Feuchtigkeitskammer
- Wiegen des Films nach der Feuchtigkeitskammer.

Die Ergebnisse zeigten, daß der Wachs/Stearinsäure/PVP-Komplex eine 1,5- bis 2,5-fache Feuchtigkeitsbeständigkeit hatte im Vergleich mit dem handelsüblichen PVP/VA Copolymer. Dies zeigt deutlich die beträchtlich erhöhte Feuchtigkeitsbeständigkeit.

Die Erfindung betrifft weiterhin ein Verfahren zur Herstellung einer feuchtigkeitsbeständigen Mascara-Zusammensetzung, das dadurch gekennzeichnet, daß man eine Ölphase, bestehend aus Wachsen oder Wachsgemischen und Stearinsäure bis zur Schmelze erhitzt, in die Schmelze bei Rührgeschwindigkeiten von 100 bis

2500 U/min ein teilchenförmiges wasserlösliches Polymeres, ausgewählt unter Polyvinylpyrrolidon, Vinylacetat/Vinylpyrrolidon-Copolymeren und Gemischen davon, in die Schmelze einträgt und bis zur Homogenität rührt,
und
a) zur Herstellung einer W/O-Emulsion in das homogene Gemisch Pigmente bis zur vollständigen Dispersion der Pigmente einbringt und eine wäßrige Phase hinzugibt, die aus Wasser und gegebenenfalls weiteren Hilfsstoffen, Trägerstoffen, Wirkstoffen und Gemischen davon besteht, und bei Temperaturen im Bereich von 60 bis 75 °C bis zum Erreichen einer homogenen Emulsion rührt, das homogene Gemisch abkühlt, oder
b) zur Herstellung einer O/W-Emulsion nach Zugabe von Pigmenten zu der wäßrigen. Phase und nach deren Dispersion die Ölphase in die wäßrige Phase einbringt und bei 60 bis 75 °C bis zum Erreichen einer homogenen Emulsion rührt und dann abkühlt.

Das erhaltene Produkt zeigt keine zwei Phasen mehr, sondern ist eine homogene Substanz, bei der thermodynamische Faktoren und oberflächenaktive Mittel (Stearinsäure) alle Partikel zusammenhalten.

Die Erfindung soll nachstehend durch das Beispiel näher erläutert werden. Alle Angeben erfolgen in Gewichtsprozent, sofern nichts anderes angegeben ist.

### Beispiel 1 Mascara

| | |
|---|---|
| **Phase A** | |
| Wasser | q.s. zu 100% |
| Konservierungsmittel | 0,3 |
| Polyquarternium-10 | 0,3 |
| Triethanolamine, 99% | 1,5 |
| Polysorbate 20 | 1,0 |
| Propylene Glycol | 4,0 |
| **Phase B** | |
| Pigmente | 10 |
| **Phase C** | |
| Synthetischer Bienenwachs | 4,0 |
| Carnaubawachs | 3,0 |
| Mikrokristalliner Wachs | 5,0 |
| Stearinsäure | 6,0 |
| Hydrogenated Castor oil | 2,0 |
| **Phase D** | |
| PVP | 3,0 |

### Verfahrensweise:

Wasser wurde auf 70-75 °C erhitzt. Die weiteren Bestandteile der Phase A wurden in der angegebenen Reihenfolge zugegeben. Danach wurde Phase B zugegeben und bis zur vollständigen Dispergierung homogenisiert. Bis zu vollständigen Klarheit wurde die Phase C geschmolzen. Die Temperatur wurde bei 75-80 °C gehalten. Die Phase D wurde zur Phase C unter mäßigem Rühren gegeben und weiter bis zur Homogenität gerührt. Danach wurde die Ölphase zur Wasserphase gegebene und emulgiert. Das Gemisch wurde auf 30 °C abgekühlt und in geeignete Behälter überführt.

## Patentansprüche

1. Feuchtigkeitsbeständige Mascara-Zusammensetzung, **gekennzeichnet durch** einen stabilen kolloidalen Komplex, bestehend aus
0,1 bis 10 Gew-% eines wasserlöslichen Polymeren, ausgewählt unter Polyvinylpyrrolidon, Vinylacetat/Vinylpyrrolidon-Copolymeren und Gemischen davon,
0,5 bis 10 Gew-% Stearinsäure und
1 bis 40 Gew-% eines Wachses oder Wachsgemisches,
und hergestellt, indem man in die Ölphase, bestehend aus dem geschmolzenen Wachs oder Wachsgemisch und Stearinsäure, das wasserlösliche Polymere oder Copolymere einbringt bis zur Bildung eines stabilen kolloidalen Komplexes, und diesen Komplex in homogener Form mit einer Wasserphase emulgiert.

2. Mascara-Zusammensetzung nach Anspruch 1, **dadurch gekennzeichnet, daß** die Wasserphase der Zusammensetzung weitere Hilfsstoffe, Trägerstoffe, Wirkstoffe oder Gemische davon enthält.

3. Verfahren zur Herstellung einer feuchtigkeitsbeständigen Mascara-Zusammensetzung, **dadurch gekennzeichnet, daß** man eine Ölphase, bestehend aus Wachsen oder Wachsgemischen und Stearinsäure bis zur Schmelze erhitzt, in die Schmelze bei Rührgeschwindigkeiten von 100 bis 2500 U/min ein teilchenförmiges wasserlösliches Polymeres, ausgewählt unter Polyvinylpyrrolidon, Vinylacetat/Vinylpyrrolidon-Copolymeren und Gemischen davon, in die Schmelze einträgt und bis zur Homogenität rührt, und
a) zur Herstellung einer W/O-Emulsion in das homogene Gemisch Pigmente bis zur vollständigen Dispersion der Pigmente einbringt und eine wäßrige Phase hinzugibt, die aus Wasser und gegebenenfalls weiteren Hilfsstoffen, Trägerstoffen, Wirkstoffen und Gemischen davon besteht, und bei Temperaturen im Bereich von 60 bis 75 °C bis zum Erreichen einer homogenen Emulsion rührt, das homogene Gemisch abkühlt, oder
b) zur Herstellung einer O/W-Emulsion nach Zugabe von Pigmenten zu der wäßrigen Phase und nach deren Dispersion die Ölphase in die wäßrige Phase einbringt und bei 60 bis 75 °C bis zum Erreichen einer homogenen Emulsion rührt und dann abkühlt.

## Claims

1. A moisture-proof mascara composition, **characterized by** a stable colloidal complex comprising
0.1 to 10 % by weight of a water-soluble polymer selected from among polyvinylpyrrolidone, vinyl acetate/vinyl pyrrolidone copolymers and mixtures thereof,
0.5 to 10 % by weight of stearic acid and
1 to 40 % by weight of a wax or wax mixture,
and manufactured by adding the water-soluble polymer or copolymer into the oil phase consisting of the melted wax or wax mixture and stearic acid, until a stable colloidal complex is formed, and emulsifying the said complex in homogeneous form with an aqueous phase.

2. Mascara composition according to claim 1, wherein the aqueous phase of the complex does contain further auxiliaries, carrier substances, active agents or mixtures thereof.

3. A method for manufacturing a moisture-proof mascara composition **characterized in that** an oil phase consisting of waxes or wax mixtures and Stearic Acid is heated until it melts, a particulate water-soluble polymer selected from among polyvinylpyrrolidone, vinyl acetate/vinyl pyrrolidone copolymers and mixtures thereof is added into the melted mass while stirring at a rate of 100 to 2500 rpm until a homogeneous mixture is obtained, and,
a) if a W/O emulsion is to be produced, pigments are added into the homogeneous mixture until they are completely dispersed, and an aqueous phase consisting of water and optionally further auxiliaries, carrier substances, active agents and mixtures thereof is added, the mixture is stirred at temperatures ranging from 60 to 75 °C until a homogeneous emulsion is obtained and the homogeneous mixture is cooled, or
b) if an O/W emulsion is to be produced, after addition of the pigments into the aqueous phase and after their dispersion, the oil phase is added into the aqueous phase and the mixture is stirred at temperatures ranging from 60 to 75 °C until a homogeneous emulsion is obtained and subsequently cooled.

## Revendications

1. Composition de mascara résistante à l'humidité, **caractérisée par** un complexe colloïdal stable, constitué par 0,1 à 10 % en poids d'un polymère hydrosoluble, choisi parmi la polyvinylpyrrolidone, les copolymères d'acétate de vinyle/vinylpyrrolidone et des mélanges de ceux-ci, 0,5 à 10 % en poids d'acide stéarique et 1 à 40 % en poids d'une cire ou d'un mélange de cires, et préparée en introduisant dans la phase huileuse, constituée par la cire ou le mélange de cires fondu(e) et l'acide stéarique, le polymère ou le copolymère hydrosoluble, jusqu'à ce qu'il se forme un complexe colloïdal stable et en émulsifiant ce complexe sous forme homogène avec une phase aqueuse.

2. Composition de mascara selon la revendication 1, **caractérisée en ce que** la phase aqueuse de la composition contient d'autres substances auxiliaires, excipients, substances actives ou des mélanges de ceux-ci.

3. Procédé de préparation d'une composition de mascara résistante à l'humidité, **caractérisé en ce qu'**on chauffe une phase huileuse, constituée par des cires ou des mélanges de cires et de l'acide stéarique jusqu'à la fusion, **en ce qu'**on introduit dans la masse fondue, à des vitesses d'agitation de 100 à 2 500 tr/min, un polymère hydrosoluble particulaire choisi parmi la polyvinylpyrrolidone, des copolymères d'acétate de vinyle/vinylpyrrolidone et des mélanges de ceux-ci et **en ce qu'**on agite jusqu'à obtenir une homogénéité, et
a) **en ce qu'**on introduit, pour la préparation d'une émulsion E/H dans le mélange homogène, des pigments jusqu'à dispersion complète des pigments et **en ce que** cela donne une phase aqueuse qui est constituée par de l'eau et le cas échéant par d'autres substances auxiliaires, excipients, substances actives et mélanges de ceux-ci et on agite à des températures dans la plage de 60 à 75 °C jusqu'à l'obtention d'une émulsion homogène et on refroidit le mélange homogène, ou
b) pour la préparation d'une émulsion H/E après avoir ajouté des pigments à la phase aqueuse et après leur dispersion, on introduit la phase huileuse dans la phase aqueuse et on agite à une température de 60 à 75 °C jusqu'à l'obtention d'une émulsion homogène et on procède ensuite à un refroidissement.
